(19) Europäisches Patentamt
European Patent Office
Office européen des brevets

(11) EP 0 729 446 B1

(12) **EUROPEAN PATENT SPECIFICATION**

(45) Date of publication and mention of the grant of the patent:
**24.02.1999 Bulletin 1999/08**

(21) Application number: **95900578.6**

(22) Date of filing: **14.11.1994**

(51) Int Cl.[6]: **C07C 15/08**, C07C 2/86

(86) International application number:
**PCT/CA94/00625**

(87) International publication number:
**WO 95/13998 (26.05.1995 Gazette 1995/22)**

(54) **PRODUCTION OF PARA-XYLENE BY SELECTIVE METHYLATION OF TOLUENE WITH METHYL HALIDES**

HERSTELLUNG VON PARA-XYLOL DURCH SELEKTIVE METHYLIERUNG VON TOLUOL MIT METHYLHALID

PRODUCTION DE PARA-XYLENE PAR METHYLATION SELECTIVE DU TOLUENE AVEC DES HALOGENURES DE METHYLE

(84) Designated Contracting States:
**AT BE CH DE DK ES FR GB GR IE IT LI LU MC NL PT SE**

(30) Priority: **15.11.1993 CA 2103116**

(43) Date of publication of application:
**04.09.1996 Bulletin 1996/36**

(73) Proprietors:
- **Gaz Métropolitain and Company, Limited Partnership**
  **Montreal, Quebec H2K 2X3 (CA)**
- **Pandey, Raj Narain**
  **Guelph, Ontario N1G 4A9 (CA)**

(72) Inventors:
- **PANDEY, Raj, Narain**
  **Guelph, Ontario N1G 4A9 (CA)**
- **RATMAMI, Kébir**
  **Boucherville, Quebec J4B 7T9 (CA)**
- **NAYAK, Vikram, Santu**
  **Guelph, Ontario N1E 6W8 (CA)**
- **FOUDA, Safaa, Abdelsamie**
  **Gloucester, Ontario K1J 6J2 (CA)**

(74) Representative: **Casalonga, Axel et al**
**BUREAU D.A. CASALONGA - JOSSE**
**Morassistrasse 8**
**80469 München (DE)**

(56) References cited:
**US-A- 4 100 215**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 729 446 B1

## Description

### TECHNICAL FIELD

The present invention relates to an improved process for the production of para-xylene. More particularly, the invention is directed to a process for the selective methylation of toluene to produce para-xylene.

### BACKGROUND ART

para-Xylene is an industrial feedstock whose demand stems mainly from its use in the production of dimethyl terephthalate (DMT) and purified terephthalic acid (PTA). DMT and PTA, in turn, are used in the production of polyester fibers and films, polyethylene terephthalate and polybutene terephthalate. para-Xylene is also used in the production of herbicides and oil additives.

para-Xylene is generally produced by reforming of naphtha to obtain xylenes-rich stream followed by separation from three close boiling $C_8$ aromatics, namely, meta-xylene, ortho-xylene and ethylbenzene. The meta- and ortho-xylenes are isomerized to obtain an equilibrium mixture of ortho-, meta- and para-xylenes and then recycled for para-xylene separation. Since the equilibrium mixture of the three xylenes contains only about 24% of para-xylene, separation and isomerization steps are repeated several times, thereby increasing the cost of para-xylene production to a relatively high level.

Another principal process for the production of xylenes is referred to as toluene disproportionation. For example, in the selective disproportionation process developed by the Mobil Chemical Company, a toluene feed and a hydrogen-rich recycle gas are reacted at elevated temperatures (>450°C) over a catalyst to produce benzene and mixed xylenes containing about 90 wt.% para-xylene. About 30 wt.% toluene is converted per pass; the unconverted toluene is separated downstream and recycled.

para-Xylene can also be produced using xylenes as feedstock. The process involves isomerization and separation of xylenes. Toluene is a preferred feedstock compared to xylenes due to its lower cost and wider availability.

The current para-xylene processes which use toluene as feedstock, such as Mobil's selective disproportionation process, suffer from major disadvantages as these processes require expensive hydrogen-rich gas, high temperatures and pressures. Processes requiring hydrogen are not only economically less attractive but also hazardous due to high pressure hydrogen.

As an alternative, methylation of toluene is an attractive and direct method of producing para-xylene. However, the development of a suitable alkylating agent and a selective catalyst is critical to successfully implementing such a process scheme. A number of studies on the methylation of toluene using methanol as an alkylating agent over a ZSM-5 type zeolite catalyst have been reported, for example, by W. W. Kaeding et al. in the Journal of Catalysis, Vol. 67, p. 159-174 (1981) and by L. B. Young et al. in the Journal of Catalysis, Vol. 76, p. 418-432 (1982). Although para-xylene is produced in good yield, the use of methanol as an alkylating agent is not economically very attractive. This is mainly because the production of methanol from coal or natural gas is a multistep process which is both energy and cost intensive, as it requires conversion of the coal or natural gas to synthesis gas by high temperature steam reforming, purification of the synthesis gas and fortification thereof with additional hydrogen, and catalytic conversion of the synthesis gas to methanol at a temperature of about 300°C and high pressures, ranging from 20,000 to 35,000 kPa (200 to 350 atm). On the other hand, if a para-xylene selectivity higher than 90% is desired, the alkylation reaction must be carried out at high temperatures (>550°C). A further drawback to utilizing methanol as an alkylating agent stems from the water which is produced as a by-product during the alkylation and which deactivates the catalyst.

US Patent N° 4,100,215 discloses a process for the selective production of para-xylene by methylation of toluene in the presence of a crystalline aluminosilicate zeolite catalyst. According to the experimental results reported in the patent, the para-xylene selectivity obtained in the methylation of toluene with methyl chloride using a zinc modified zeolite catalyst is not more than 50%. Even lower para-xylene selectivities were obtained when using methanol as methylating agent and conducting the methylation in the presence of an unmodified, protonated zeolite catalyst.

### DISCLOSURE OF THE INVENTION

It is therefore an object of the present invention to overcome the above drawbacks and to provide a process for the production of para-xylene with high selectivity, at less severe conditions of temperature and pressure than those encountered in the prior art.

In accordance with the invention, there is thus provided a process for the selective methylation of toluene to produce para-xylene, which comprises reacting toluene with a methyl halide at a temperature ranging from 100 to 500°C and a pressure ranging from 100 to 1000 kPa (1 to 10 atm), in the presence of a heteropolyacid modified, shape-selective zeolite catalyst in protonated form having a Si/Al ratio of at least 25 and less than 50.

The methylation of toluene with methyl halide generally yields a mixture of xylenes, according to the following equation:

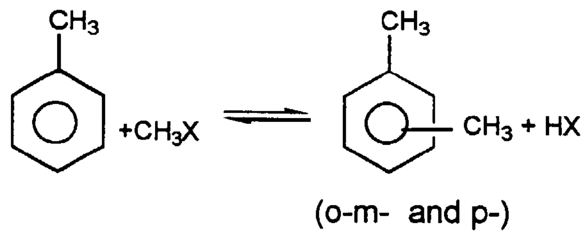


wherein X is a halogen atom. As the thermodynamic equilibrium concentrations of ortho-, meta- and para-xylenes are in ratio of 1 : 2 : 1, Applicant has found quite unexpectedly that the more useful para-xylene can be selectively produced at temperatures less than 500°C by using a shape selective aluminosilicate catalyst such as a ZSM-5 type zeolite, provided that such a zeolite catalyst be in protonated form and have a Si/Al ratio of at least 25 and less than 50, and that it be modified with a heteropoly acid. The shape selective behaviour of ZSM-5 zeolite is attributed to its unique three dimensional network of elliptical straight channels and near circular zigzag channels. Since the critical diameter of para-xylene molecules is smaller than ortho- or meta-xylene molecules, para-xylene diffuses much faster than ortho or meta-xylene inside the protonated ZSM-5 zeolite channels. Consequently, the equilibrium mixture of xylenes inside the zeolite channels gets depleted in para-xylene, as more and more para-xylene diffuses out of the channels. As most of the acid sites are located inside the zeolite channels, the ortho- and meta-xylenes quickly undergo isomerization to produce para-xylene in order to maintain the equilibrium concentration. The reverse reaction, that is, the isomerization of para-xylene to produce undesirable ortho- and meta-xylenes, occurs to a very small extent due to the limited active sites on the external surface of the zeolite crystals. This leads to a product mixture rich in para-xylene.

Protonation of the zeolite catalyst activates the catalyst for the selective alkylation of toluene with the methyl halide. Preferably, from 10 to 100% of the $Na^+$ ions contained in the zeolite catalyst are replaced with $H^+$ ions. The degree of $H^+$ exchange, i.e.

$$\frac{[H^+]}{[H^+]+[Na^+]} \times 100,$$

determines the number of protons associated with the aluminum atoms in the zeolite. Protonation can be carried out by impregnating the catalyst with an aqueous solution of HCl or other mineral acid, or with an aqueous solution of $NH_4NO_3$, followed by calcination at temperatures of 400-500°C for a period of about 4 hours. The ZSM-5 zeolite, on the other hand, can be prepared by the method described in US Patent No. 3,702,886.

Examples of suitable heteropoly acids which may be used for modifying the zeolite catalyst include 12-tungstophosphoric acid ($H_3PW_{12}O_{40}$), 12-tungstosilicic acid ($H_4SiW_{12}O_{40}$) and 12-molybdophosphoric acid ($H_3PMO_{12}O_{40}$). These acids have a high thermal stability, high solubility in water and high Brönsted acidity. Treatment is effected by impregnating the catalyst with an aqueous solution of the heteropoly acid and thereafter evaporating the water. The heteropoly acid deposited on the catalyst remains solid at the operating temperatures, i.e. up to 500°C. Preferably, the modified zeolite catalyst comprises from 0.5 to 25 wt.% of heteropoly acid.

Modification of the protonated zeolite catalyst with a heteropoly acid has been found to increase the para-xylene selectivity in the case where the catalyst used has a Si/Al ratio less than 50. It is believed that the increased selectivity for para-xylene is due to the heteropoly acid blocking the active sites on the outer surface of the catalyst which are responsible for side reactions leading to the formation of trimethylbenzenes and higher aromatic hydrocarbons.

The methyl halide which is used as alkylating agent can be methyl chloride, methyl bromide or methyl iodide. Where use is made of methyl chloride, such an alkylating agent is preferably produced by chlorination of methane or natural gas. Commercial scale production of methyl chloride is usually carried out by thermal chlorination of methane, also called oxyhydrochlorination, at temperatures of 300-450°C. The reaction is highly selective to methyl chloride. For example, using a silica-supported $CuCl-KCl-LaCl_3$ catalyst at 340°C, and a reactant mixture of 40% $CH_4$, 40% HCl and 20% $O_2$, methyl chloride selectivity in the range of 60-85% at methane conversion in the range of 18-43% has been reported by C.E. Taylor et al in "Methane Conversion", Studies in Surface Science Catalysis, Vol 36, pages 483-489 (1988). The chlorination of methane can also be carried out by photochemical methods, wherein a mixture of

methane and chloride is exposed to an ultraviolet radiation with a wavelength in the region 250 to 500 nm at low temperature.

The methylation of toluene with the methyl halide over the protonated zeolite catalyst is preferably carried out at a temperature of 300 to 400°C, with a weight hourly space velocity of the reactants ranging from 0.01 to 10 $h^{-1}$, preferably from 0.1 to 1 $h^{-1}$. The toluene/methyl halide ratio generally ranges from 0.1 to 10.

Where use is made of natural gas-derived methyl chloride, the invention provides an economical route for producing para-xylene with high selectivity, while utilizing abundantly available natural gas resources.

BRIEF DESCRIPTION OF THE DRAWINGS

Figure 1 is a flow diagram of a process for producing para-xylene according to the invention.

MODES OF CARRYING OUT THE INVENTION

In the process which is schematically illustrated in Fig. 1, para-xylene is produced by the selective methylation of toluene with natural gas-derived methyl chloride. Oxyhydrochlorination of natural gas is carried out in a first stage reactor 10 to produce methyl chloride which is used for selectively methylating toluene in a second stage reactor 12. Natural gas, hydrogen chloride and air are fed via feed lines 14, 16 and 18, respectively, to reactor 10 containing a fixed bed of silica-supported $CuCl-KCl-LaCl_3$ catalyst maintained at about 350°C. The product of reaction which is discharged via line 20 and comprises a mixture of methyl chloride, other chloromethanes, unreacted hydrogen chloride and water is passed through a heat exchanger 22 for lowering the temperature from about 350°C to about 20°C, and then sent to a separator 24 for separating the methyl chloride from the unreacted hydrogen chloride, air and the water which are discharged as by-products via line 26. The hydrogen chloride discharged via line 26 can be recycled as a feedstock to the reactor 10. The separated methyl chloride is sent via line 28 to the reactor 12 where it is reacted with toluene fed through line 30. The reactor 12 contains a fixed bed of protonated ZSM-5 zeolite catalyst maintained at a temperature of about 375°C. The product of reaction which is discharged via line 32 and comprises a mixture of $C_2$-$C_4$ hydrocarbons, aromatics, hydrogen chloride and unreacted toluene is passed through a heat exchanger 34 for lowering the temperature from about 375°C to about 20°C, and then sent to a stripper column 36. The $C_2$-$C_4$ hydrocarbons are stripped off in the stripper 36 and discharged via line 38 for storage or consumption as gaseous fuel. The hydrogen chloride which is recovered as a by-product is recycled via line 40 to the first stage reactor 10. The remaining aromatics which are removed at the bottom of the stripper column 36 are sent via line 42 to a distillation column 44 for recovery of the unreacted toluene and separation of the $C_8$ aromatics. The lighter toluene fraction is recycled via line 46 to the second stage reactor 12. Benzene obtained as a by-product is discharged via line 48. The heavier xylene fraction containing small quantities of ethylbenzene and $C_9$ aromatics is removed at the bottom of the distillation column 44 and sent via line 50 to a mixed xylenes separation unit (not shown) for the extraction of para-xylene.

The following non-limiting example further illustrates the invention.

**EXAMPLE**

Methylation of toluene with methyl chloride was also carried out using a protonated ZSM-5 zeolite catalyst modified with 10 wt.% of 12-tungstophosphoric acid and having a Si/Al ratio of 36. The modified zeolite catalyst was tested for its activity and selectivity in the methylation of toluene at 350°C and atmospheric pressure. The activity and product selectivity of the modified zeolite catalyst were compared with those of an unmodified, protonated ZSM-5 zeolite catalyst having the same Si/Al ratio. A comparison of the product distributions obtained with the two catalysts at 350°C is shown in the following Table.

TABLE

| Si/Al ratio | 36 (modified) | 36 (unmodified) |
|---|---|---|
| Time on Stream (minutes) | 180 | 185 |
| WHSV ($h^{-1}$) | | |
| - Methyl Chloride | 0.32 | 0.32 |
| - Toluene | 0.59 | 0.59 |
| Conversion (%) | | |
| - Methyl Chloride | 66.8 | 80.3 |

TABLE (continued)

| | | |
|---|---|---|
| - Toluene | 24.9 | 34.4 |
| Hydrocarbon Distribution (wt.%) | | |
| $C_1$-$C_4$ Aliphatics | 17.9 | 14.9 |
| $C_{5+}$ Aliphatics | 1.0 | 0.5 |
| Benzene | 0.4 | 0.5 |
| Ethylbenzene | 0.2 | 0.5 |
| p-Xylene | 24.5 | 18.5 |
| m-Xylene | 20.9 | 24.2 |
| o-Xylene | 8.8 | 10.5 |
| 3-Methylethylbenzene | 3.7 | 3.1 |
| 4-Methylethylbenzene | 4.5 | 5.1 |
| 2-Methylethylbenzene | 0.2 | 0.3 |
| 1,3,5-Trimethylbenzene | 0.2 | 0.4 |
| 1,2,4-Trimethylbenzene | 17.1 | 20.2 |
| 1,2,3-Trimethylbenzene | 0.0 | 1.1 |
| $C_{10}$ Aromatics | 0.5 | 0.2 |
| Total Xylenes | 54.2 | 53.2 |
| Isomer distribution (%) | | |
| p-xylene | 45.2 | 34.8 |
| m-xylene | 38.6 | 45.5 |
| o-xylene | 16.2 | 19.7 |
| Total Ethyltoluenes | 8.4 | 8.5 |
| Total Trimethylbenzenes | 17.3 | 21.7 |

**Claims**

**1.** A process for the selective methylation of toluene to produce para-xylene, which comprises reacting toluene with a methyl halide at a temperature ranging from 100 to 500°C and a pressure ranging from 100 to 1000 kPa (1 to 10 atm), in the presence of a heteropolyacid modified, shape-selective zeolite catalyst in protonated form having a Si/Al ratio of at least 25 and less than 50.

**2.** A process as claimed in claim 1, wherein said methyl halide is selected from the group consisting of methyl chloride, methyl bromide and methyl iodide.

**3.** A process as claimed in claim 2, wherein said methyl halide is methyl chloride.

**4.** A process as claimed in claim 3, wherein said methyl chloride is produced by chlorination of methane or natural gas.

**5.** A process as claimed in claim 4, wherein said methyl chloride is produced by oxyhydrochlorination of natural gas.

**6.** A process as claimed in claim 1, wherein said reaction is carried out at a temperature of 300 to 400°C.

**7.** A process as claimed in claim 1 or 6, wherein said reaction is carried out at atmospheric pressure.

**8.** A process as claimed in claim 1, wherein the toluene and methyl halide are reacted in a ratio of toluene/methyl halide ranging from 0.1 to 10.

**9.** A process as claimed in claim 8, wherein said toluene/methyl halide ratio is 1.

**10.** A process as claimed in claim 1, wherein the reaction is carried out utilizing a weight hourly space velocity of each of toluene and methyl halide in the range of 0.01 to 10 $h^{-1}$.

**11.** A process as claimed in claim 10, wherein said weight hourly space velocity ranges from 0.1 to 1 $h^{-1}$.

**12.** A process as claimed in claim 1, wherein said zeolite catalyst is modified with a heteropoly acid selected from the group consisting of 12-tungstophosphoric acid, 12-tungstosilicic acid and 12-molybdophosphoric acid.

**13.** A process as claimed in claim 12, wherein the modified zeolite catalyst comprises from 0.5 to 25 wt.% of said heteropoly acid.

**14.** A process as claimed in claim 13, wherein said modified zeolite catalyst comprises 10 wt.% of 12-tungstophosphoric acid.

**15.** A process as claimed in claim 12, 13, or 14, wherein said methyl halide is methyl chloride produced by chlorination of natural gas.

**Patentansprüche**

**1.** Verfahren zur selektiven Methylierung von Toluol zum Herstellen von para-Xylol, das umfaßt Umsetzen von Toluol mit einem Methylhalogenid bei einer Temperatur im Bereich von 100 bis 500 °C und einem Druck im Bereich von 100 bis 1000 kPa (1 bis 10 atm), in Gegenwart eines mit Heteropolysäure modifizierten, formselektiven Zeolithkatalysators in protonierter Form mit einem Si/Al-Verhältnis von mindestens 25 und weniger als 50.

**2.** Verfahren nach Anspruch 1, worin das Methylhalogenid ausgewählt ist aus der Gruppe bestehend aus Methylchlorid, Methylbromid und Methyliodid.

**3.** Verfahren nach Anspruch 2, worin das Methylhalogenid Methylchlorid ist.

**4.** Verfahren nach Anspruch 3, worin das Methylchlorid durch Chlorierung von Methan oder Erdgas produziert wird.

**5.** Verfahren nach Anspruch 4, worin das Methylchlorid durch Oxyhydrochlorierung von Erdgas produziert wird.

**6.** Verfahren nach Anspruch 1, worin die Reaktion bei einer Temperatur von 300 bis 400 °C ausgeführt wird.

**7.** Verfahren nach Anspruch 1 oder 6, worin die Reaktion bei Atmosphärendruck ausgeführt wird.

**8.** Verfahren nach Anspruch 1, worin das Toluol und Methylhalogenid in einem Verhältnis von Toluol/Methylhalogenid im Bereich von 0,1 bis 10 umgesetzt werden.

**9.** Verfahren nach Anspruch 8, worin das Verhältnis von Toluol/Methylhalogenid 1 beträgt.

**10.** Verfahren nach Anspruch 1, worin die Reaktion ausgeführt wird unter Anwendung einer stundenbezogenen Masse-Raumgeschwindigkeit im Bereich von 0,01 bis 10 $h^{-1}$ sowohl für Toluol wie für Methylhalogenid.

**11.** Verfahren nach Anspruch 10, worin die stundenbezogene Masse-Raumgeschwindigkeit im Bereich von 0,1 bis 1 $h^{-1}$ liegt.

**12.** Verfahren nach Anspruch 1, worin der Zeolithkatalysator mit einer Heteropolysäure ausgewählt aus der Gruppe bestehend aus 12-Wolframatophosphorsäure, 12-Wolframatokieselsäure und 12-Molybdatophosphorsäure modifiziert ist.

**13.** Verfahren nach Anspruch 12, worin der modifizierte Zeolithkatalysator von 0,5 bis 25 Gew.-% der Heteropolysäure enthält.

**14.** Verfahren nach Anspruch 13, worin der modifizierte Zeolithkatalysator 10 Gew.-% 12-Wolframatophosphorsäure enthält.

15. Verfahren nach Anspruch 12, 13 oder 14, worin das Methylhalogenid durch Chlorierung von Erdgas produziertes Methylchlorid ist.

## Revendications

1. Procédé de méthylation sélective du toluène, produisant du para-xylène, qui comporte le fait de faire réagir du toluène avec un halogénure de méthyle à une température de 100 à 500 °C et sous une pression de 100 à 1000 kPa (1 à 10 atm), en présence d'un catalyseur morphosélectif qui est une zéolite modifiée par un hétéropolyacide et se présentant sous forme protonée, chez laquelle le rapport Si/Al vaut au moins 25 et moins de 50.

2. Procédé conforme à la revendication 1, dans lequel ledit halogénure de méthyle est choisi dans l'ensemble constitué par le chlorure de méthyle, le bromure de méthyle et l'iodure de méthyle.

3. Procédé conforme à la revendication 1, dans lequel ledit halogénure de méthyle est du chlorure de méthyle.

4. Procédé conforme à la revendication 3, dans lequel ledit chlorure de méthyle est produit par chloration de méthane ou de gaz naturel.

5. Procédé conforme à la revendication 4, dans lequel ledit chlorure de méthyle est produit par oxychloration de gaz naturel.

6. Procédé conforme à la revendication 1, dans lequel ladite réaction se déroule à une température de 300 à 400 °C.

7. Procédé conforme à la revendication 1 ou 6, dans lequel ladite réaction est effectuée sous la pression atmosphérique.

8. Procédé conforme à la revendication 1, dans lequel on fait réagir le toluène et l'halogénure de méthyle en un rapport toluène/(halogénure de méthyle) valant de 0,1 à 10.

9. Procédé conforme à la revendication 8, dans lequel ledit rapport toluène/(halogénure de méthyle) vaut 1.

10. Procédé conforme à la revendication 1, dans lequel on effectue la réaction avec une vitesse spatiale horaire massique valant de 0,01 à 10 $h^{-1}$ pour chacun des réactifs toluène et halogénure de méthyle.

11. Procédé conforme à la revendication 10, dans lequel ladite vitesse spatiale horaire massique vaut de 0,1 à 1 $h^{-1}$.

12. Procédé conforme à la revendication 1, dans lequel ladite zéolite servant de catalyseur est modifiée par un hétéropolyacide choisi dans l'ensemble constitué par l'acide phosphoduodécitungstique, l'acide silicoduodécitungstique et l'acide phosphoduodécimolybdique.

13. Procédé conforme à la revendication 12, dans lequel la zéolite modifiée servant de catalyseur contient de 0,5 à 25 % en poids dudit hétéropolyacide.

14. Procédé conforme à la revendication 13, dans lequel ladite zéolite modifiée servant de catalyseur contient 10 % en poids d'acide phosphoduodécitungstique.

15. Procédé conforme à la revendication 12, 13 ou 14, dans lequel ledit halogénure de méthyle est du chlorure de méthyle produit par chloration de gaz naturel.

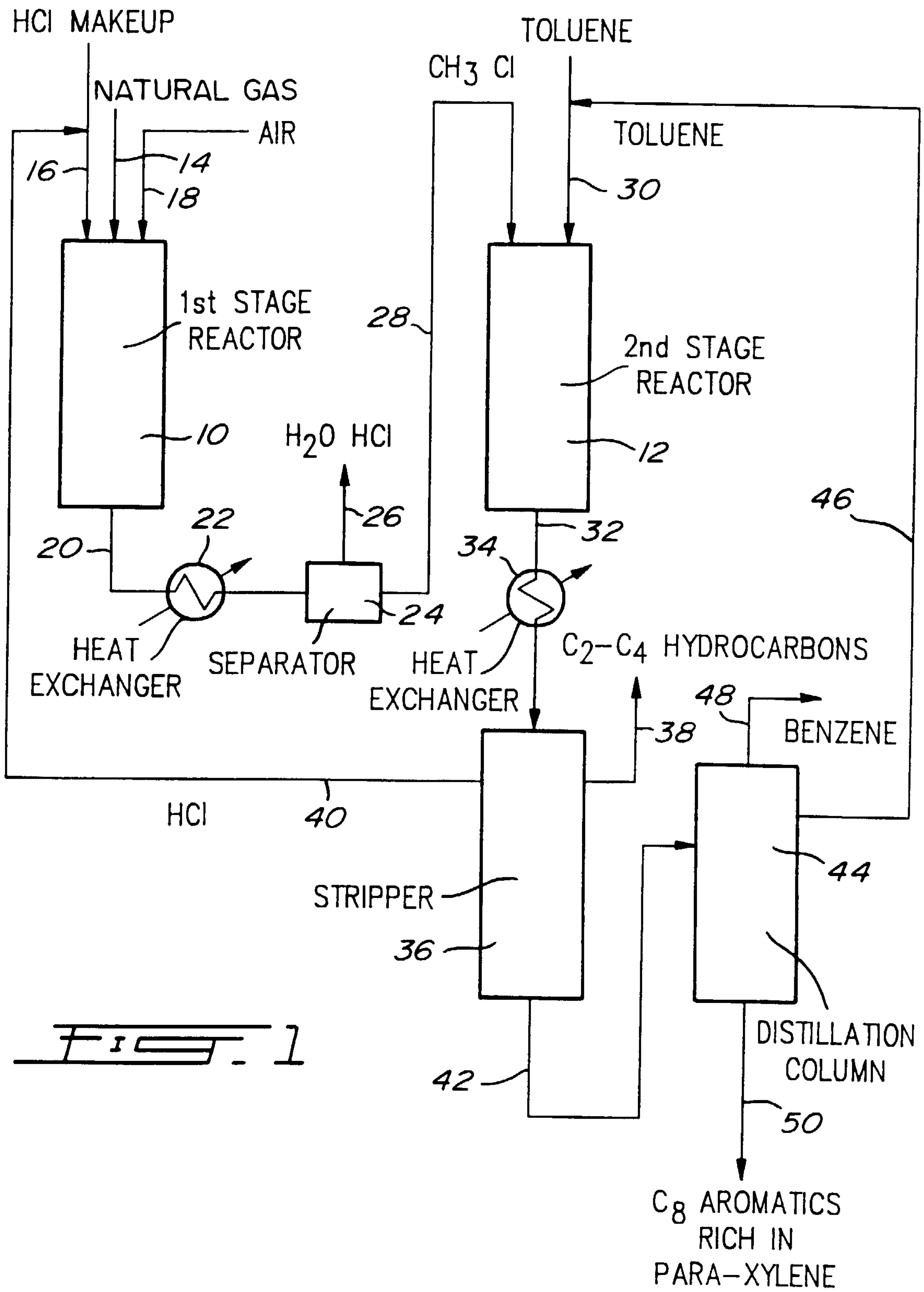

HCl MAKEUP
NATURAL GAS
AIR
16
14
18
1st STAGE REACTOR
10
20
22
HEAT EXCHANGER
SEPARATOR
24
26
H2O HCl
28
CH3 Cl
TOLUENE
TOLUENE
30
2nd STAGE REACTOR
12
32
34
HEAT EXCHANGER
C2-C4 HYDROCARBONS
38
HCl
40
STRIPPER
36
42
48
BENZENE
44
DISTILLATION COLUMN
46
50
C8 AROMATICS RICH IN PARA-XYLENE


FIG. 1